(19) Europäisches Patentamt
European Patent Office
Office européen des brevets

(11) Publication number: **0 154 432 B1**

# (12) EUROPEAN PATENT SPECIFICATION

(45) Date of publication of patent specification: **11.04.90**

(51) Int. Cl.$^5$: **C 12 N 11/08**, A 61 K 37/547

(21) Application number: **85301032.0**

(22) Date of filing: **15.02.85**

## (54) Modified urokinase composition.

(30) Priority: **21.02.84 JP 29499/84**

(43) Date of publication of application:
**11.09.85 Bulletin 85/37**

(45) Publication of the grant of the patent:
**11.04.90 Bulletin 90/15**

(84) Designated Contracting States:
**CH DE FR GB IT LI**

(56) References cited:
**EP-A-0 098 110**
**DE-A-3 033 030**
**FR-A-2 515 684**

**CHEMICAL ABSTRACTS, vol. 99, no. 5, 1st August 1983, page 284, abstract no. 35607z, Columbus, Ohio, US; C.O. BEAUCHAMP et al.: "A new procedure for the synthesis of polyethylene glycol-protein adducts; effects on function, receptor recognition, and clearance of superoxide dismutase, lactoferrin, and alpha2-macroglobulin", & ANAL. BIOCHEM. 1983, 131(1), 25-33**

(73) Proprietor: **NIHON CHEMICAL RESEARCH KABUSHIKI KAISHA also known as JAPAN CHEMICAL RESEARCH CO., LTD**
**4-20, Mikagehommachi 3-chome Higashinada-ku**
**Kobe Hyogo 658 (JP)**

(73) Proprietor: **KABUSHIKI KAISHA SANWA KAGAKU KENKYUSHO also known as SANWA KAGAKU KENKYUSHO CO., LTD.**
**No. 35, Higashisotobori-cho**
**Higashi-ku Nagoya-shi Aichi 461 (JP)**

(72) Inventor: **Sawai, Kiichi**
**36-14, Ninomiya 1-chome**
**Funabashi Chiba 274 (JP)**
Inventor: **Kurono, Masayasu**
**7-17, Kakeage 1-chome**
**Minami-ku Nagoya Aichi 457 (JP)**
Inventor: **Awaya, Juichi**
**1-111, Amakoda**
**Moriyama-ku Nagoya Aichi 463 (JP)**
Inventor: **Iwata, Tadahiko**
**760-763, Sakashita-cho 7-chome**
**Kasugai Aichi 480-03 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 154 432 B1

(74) Representative: Burford, Anthony Frederick et al
W.H. Beck, Greener & Co. 7 Stone Buildings
Lincoln's Inn
London WC2A 3SZ (GB)

## Description

This invention relates to a novel modified urokinase composition. Thrombi appears to rank high on the list of causes of death when cerebral thrombosis, cardiac infarction, pulmonary infarction, etc., which account for an increasing proportion of vascular disturbance, are taken into account. The incidence of thrombosis is expected to increase further in view of the increasing habit of taking Western-style diets and the increasing proportion of aged population due to an extension of mean life. For the treatment of thrombosis by way of activation of the fibrinolytic system, plasminogen-activating substances are commonly administered. Of such plasminogen activators, those which are readily available on the market in quantities are urokinase and streptokinase, but since streptokinase is a heterogenous protein derived from β-hemolytic streptococci and, as such, is antigenic, urokinase is in common use in Japan. Urokinase is a human-derived enzyme and is known to exist in two types, namely the high molecular urokinase (mol. wt. 54000) and the low molecular urokinase (mol. wt. 33000). However, the blood half-life of urokinase administered is only a few minutes and the elimination is too fast for expected enzymatic activity to be realized. It has also been shown that since urokinase administered is inactivated by a urokinase inhibitor in the blood, its thrombolytic function is not implemented unless it is administered in a dose beyond a certain threshold level. Because urokinase is a substance showing such a haemokinetic characteristic, the treatment of thrombosis with urokinase has to depend on the use of massive doses.

DE—A—3033030 (corresponding to US—A—4349630) discloses urokinase derivatives of copolymers of acrylamide and acrylic acid prepared using 1-ethyl-3-(3-dimethylaminopropyl) carbodiimide as an activator.

EP—A—0098110 discloses polypeptide or glycoprotein derivatives of monomethoxy-polyoxyethylene-polyoxypropylene copolymers in which the terminal hydroxyl group of the copolymer is linked to the amino group of the polypeptide or glycoprotein by a coupling agent. The specified coupling agents include dibromosuccinic anhydride and maleic anhydride but there is no exemplification of the use of either of these anhydrides with urokinase. The only example (Example 1) of a urokinase derivative uses cyanuric chloride (i.e. 2,4,6-trichloro-s-triazine) as the coupling agent.

It is a primary object of this invention to improve the stability of urokinase in the blood so that the duration of its action as well as its efficacy as a thrombolytic agent may be increased. The specific object of this invention is to provide a modified urokinase composition which is physicochemically stable and less liable to the influence of he urokinase inhibitor in the blood and, therefore, with a remarkably increased blood half-life; a method of producing such modified urokinase composition; and a thrombolytic composition containing the same. In accordance with this invention, these objects are accomplished with a modified urokinase composition of the formula:

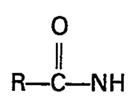 urokinase,

wherein R is the residue of a water-soluble monomethoxypolyethylene or polyethylene-polypropylene glycol with a molecular weight of 2000 to 10000 after removal of hydrogen from the terminal hydroxyl group.

The modified urokinase composition of the above formula is a novel composition, and can be produced in accordance with this invention by reacting a homo- or co-polymer carbonylimidazole derivative of the formula

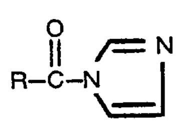 ,

wherein R has the same meaning as defined hereinbefore, with urokinase in the presence of a buffer solution at pH 6 to 11.

In this reaction, the hydrogen atoms in the amino groups, i.e. the ε-amino group of the lysine unit and the N-terminal amino group, of the urokinase molecule are substituted by

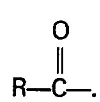.

The homopolymer or copolymer mentioned above is monomethoxy-polyethylene glycol (PEG) or monomethoxy-polyethylene glycol-polypropylene glycol (PEG·PPG). The carbonylimidazole derivative of such polymer or copolymer (CDI—PEG or CDI—PEG·PPG) which is employed in accordance with this invention can be produced by activating the hydroxy groups of said glycol with 1,1′-carbonyldiimidazole in

the conventional manner (Charles O. Beauchamp et al, Analytical Biochemistry 131, 25—33 (1983)), i.e. by adding to a dioxane solution of PEG or PEG·PPG 10 times its amount of 1,1'-carbonyldiimidazole, allowing the mixture to react at 37°C with stirring for 2 hours, dialyzing the reaction mixture against distilled water urokinase preparations. The modified urokinase composition according to this invention is, therefore, of remarkable value as a pharmaceutical product.

In the acute toxicity study of the modified urokinase composition of this invention in mice and rats, dose ($1.8 \times 10^5$ IU/kg) equivalent to about 1500 times the ordinary dose to humans (120 IU/kg) were administered orally, subcutaneously and intravenously. In these experiments, neither deaths or abnormal changes in general condition were encountered. Therefore, the $LD_{50}$ values of the modified urokinase composition according to this invention by any administration route are more than $1.8 \times 10^5$ IU/kg, indicating that the safety of the composition is very high.

The modified urokinase composition according to this invention can be processed into any desired dosage forms such as parenteral products, oral preparations, etc.

This invention will hereinafter be illustrated in further detail, referring particularly to the production method. The "GGA—MCA method" used in the examples for assay or urokinase activity means the Glt—Gly—Arg—MCA hydrolytic activity assay; the "plate method" is the standard fibrin plate method comprising measuring the area of the lysis window; and the "test tube method" is the method comprising measuring the time till a ball falling through a mass of lyzed fibrin in a test tube reaches the tube bottom.

Referring to the accompanying drawings:

Fig. 1 is a graph showing the relation of the concentration of monomethoxy-polyethylene glycol carbonylimidazole derivative used as the modifier with the residual activity of the modified urokinase;

Fig. 2 is a graph showing the relation of the reaction time between urokinase and monomethoxypolyethylene glycol carbonylimidazole derivative used as the modifier at the concentration of the latter of 4 mM with the residual activity of the modified urokinase;

Fig. 3 is a graph similar to Fig. 1, showing the relation of the concentration of modifier monomethoxypolyethylene glycol-propylene glycol carbonylimidazole derivative with the residual activity of the modified urokinase;

Fig. 4 is a graph showing the resistance of monomethoxy-polyethylene glycol-modified urokinase to a urokinase inhibitor;

Fig. 5 is a graph similar to Fig. 4, showing the resistance of modified urokinase as modified by monomethoxy-ethylene glycol-polypropylene glycol derivative; and

Fig. 6 is a graph showing the time course of blood urokinase activity after intravenous injection of the modified urokinase of this invention into rabbits.

## Production Example 1

Production of monomethoxy-polyethylene glycol-modified urokinase composition: $2 \times 10^{-2}$ mM of a high molecular weight urokinase (about 100000 IU/ml) was reacted with varying concentrations of monomethoxy-polyethylene glycol carbonylimidazole derivative with an average molecular weight of 5000 (1—16 mM of CDI—PEG) in 10 mM borate buffer (pH 8.5) under stirring at 4°C for 42 hours. The reaction mixture was dialyzed to remove the unreacted excess reagent and made up to 5 ml with physiological saline solution to give the desired modified urokinase composition.

## Test Example 1

The unmodified urokinase activity was determined by the test tube method, plate method and GGA—MCA method to investigate the relation of activity with the concentration of CDI—PEG (0, 1, 2, 4, 6 and 16 mM). The results are shown in Fig. 1. The activity values found by the test tube method are 15987, 15840, 11132, 7084, 4732 and 1610 IU/ml. Therefore, the preferred concentration of CDI—PEG for pharmaceutical purposes was about 4 mM. Accordingly, the concentration of CDI—PEG was set at 4 mM and the relation of reaction time was residual urokinase activity was investigated. The results are shown in Fig. 2. Thus, the preferred reaction time was about 40 hours.

## Production Example 2

Production of monomethoxy-polyethylene glycol-polypropylene glycol-modified urokinase composition: $2 \times 10^{-2}$ mM of a high molecular weight urokinase (about 100000 IU/ml) was reacted with varying concentrations of monomethoxy-polyethylene glycol-polypropylene glycol carbonylimidazole derivative with an average molecular weight of 5,000 (1—32 mM of CDI—PEG·PPG) in 10 mM borate buffer (pH 8.5) under stirring at 4°C for 42 hours. The reaction mixture was dialyzed to remove the excess unreacted reagent and made up to 5 ml with physiological saline solution to give the desired modified urokinase composition.

## Test Example 2

As in Test Example 1, the activities of unmodified urokinase and various modified urokinases according to Production Example 2 were assayed by the test tube method, plate method and GGA—MCA method to investigate the relation of activity with the concentration (0, 1, 4, 8, 16 and 32 mM) of CDI—PEG·PPG. The results are shown in Fig. 3. The activity values found by the test tube method were

17093, 8450, 4120, 2620, 1500 and 517 IU/ml. Thus, the preferred concentration of CDI—PEG·PPG for pharmaceutical purposes was about 4 mM.

Test Example 3

For the purpose of investigating the resistance of modified urokinase compositions according to Production Examples 1 and 2 (prepared using 4 mM each of CDI—PEG and CDI—PEG·PPG) to an urokinase inhibitor (placental origin), the concentration of the inhibitor was varied (over 2.5 through 160 IU/ml) and the residual activity was assayed. The results are shown in Figs. 4 and 5, respectively.

It is found from Figs. 4 and 5 that the modified urokinase composition according to this invention has a high resistance to the inhibitor as compared with unmodified urokinase.

Test Example 4

Each of the modified urokinase compositions according to Production Examples 1 and 2 (prepared using 4 mM each of CDI—PEG and CDI—PEG·PPG, respectively) was intravenously administered to rabbits in the dose of 50,000 IU/kg body weight and the time course of blood urokinase activity was investigated by the GGA—MCA assay method. The results are shown in Fig. 6.

In view of the fact that the elimination half-time of urokinase in the blood is generally a few minutes, it is clear from Fig. 6 that the modified urokinase composition according to this invention has a remarkably high stability in the blood.

Test Example 5

Stability of the modified urokinase of this invention in solution:

The enzymatic activity (potency) values of the modified urokinase compositions according to Production Examples 1 and 2 after lyophilization were as shown in the following table.

| | | GGA+MCA assay | Test tube assay | Plate assay |
|---|---|---|---|---|
| CDI—PEG—UK | Albumin | + 91298 (IU/ml) | 20350 (IU/ml) | 19863 (IU/ml) |
| | | − 93606 | 19875 | 19178 |
| CDI—PEG · PPG—UK | Albumin | + 99159 | 21917 | 24702 |
| | | − 98261 | 21567 | 28846 |

Each of the lyophilized modified urokinase compositions was dissolved in 1 ml of physiological saline, and after standing at 37°C for 24 hours, the potency was determined. The results are set forth in the following table.

| | | GGA+MCA assay | Test tube assay | Plate assay |
|---|---|---|---|---|
| CDI—PEG—UK | Albumin | + 93822 (IU/ml) | 19720 (IU/ml) | 20514 (IU/ml) |
| | | − 93662 | 21000 | 19775 |
| CDI—PEG · PPG—UK | Albumin | + 95552 | 23227 | 25666 |
| | | − 102955 | 18827 | 24113 |

It will be apparent from the above results that the modified urokinase according to this invention has a fairly high stability in solution as well.

Preparation Example

To 1000 IU of the modified urokinase composition according to Production Example 1 was added an appropriate amount of albumin, dextran or gelatin, followed by addition of 2.86 mg of monosodium phosphate, 13.2 mg of disodium phosphate and 38.5 mg of D-mannitol. The mixture was lyophilized. This lyophilisate is extemporaneously dissolved in 1 ml of distilled water for injection and the solution is used as an injection.

This injection has a pH value of 7.0 ± 0.5 and an osmotic pressure of about 1 relative to 0.9% physiological saline solution.

## Claims

1. A modified urokinase in which a water-soluble polymer or co-polymer is coupled to the amine group of the urokinase, characterized in that the modified urokinase has the formula

R.CO.NH.urokinase

wherein R is the residue of a water-soluble monomethoxy-polyethylene glycol or monomethoxy polyethylene glycol-polypropylene glycol with a molecular weight of 2,000 to 10,000 after removal of hydrogen from the terminal hydroxyl group.

2. A modified urokinase as claimed in Claim 1, wherein the moiety R has an average molecular weight of 5,000.

3. A modified urokinase as claimed in Claim 1 or Claim 2 having a molecular weight in the range of 100,000 to 150,000 and modified to a residual activity of 20 to 40%.

4. A modified urokinase as claimed in any one of the preceding Claims for use in the treatment of thrombosis.

5. A method of producing a modified urokinase as claimed in any one of the preceding Claims which comprises reacting a homo- or co-polymer carbonylimidazole derivative of the formula

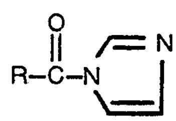,

wherein R is as defined in Claim 1, with urokinase in the presence of a buffer solution as pH 6 to 11.

6. A thrombolytic composition containing a modified urokinase as claimed in any one of Claims 1 to 3 in admixture, or otherwise associated, with a pharmaceutically acceptable diluent or carrier.

7. The use of a modified urokinase as claimed in any one of Claims 1 to 3 for preparing a thrombolytic composition.

## Patentansprüche

1. Modifizierte Urokinase, bei der ein wasserlösliches Polymer oder Copolymer an die Aminogruppe der Urokinase gekoppelt ist, dadurch gekennzeichnet, daß die modifizierte Urokinase die Formel

R.CO.NH. Urokinase

aufweist, in der R der Rest eines wasserlöslichen Monomethoxypolyethylenglykols oder Monomethoxypolyethylenglykolpolypropylenglykols mit einem Molekulargewicht von 2000 bis 10 000 nach Entfernung des Wasserstoffs von der terminalen Hydroxylgruppe ist.

2. Modifizierte Urokinase nach Anspruch 1, dadurch gekennzeichnet, daß der Rest R ein durchschnittliches Molekulargewicht von 5000 hat.

3. Modifizierte Urokinase nach Anspruch 1 oder Anspruch 2 mit einem Molekulargewicht im Bereich von 100 000 bis 150 000 und modifiziert bis zu einer Restaktivät von 20 bis 40%.

4. Modifizierte Urokinase gemäß jeglichem der vorangehenden Ansprüche zur Verwendung bei der Behandlung von Thrombose.

5. Verfahren zur Herstellung einer modifizierten Urokinase gemäß jeglichem der vorangehenden Ansprüche, dadurch gekennzeichnet, daß man ein homo- oder copolymeres Carbonylimidazol-Derivat der Formel

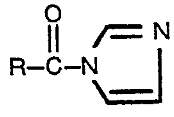,

in der R gemäß Anspruch 1 definiert ist, in Gegenwart einer Pufferlösung bei pH 6 bis 11 mit Urokinase umsetzt.

6. Eine thrombolytische Zusammensetzung, die eine modifizierte Urokinase gemäß jeglichem der Ansprüche 1 bis 3 in Mischung oder in sonstiger Weise verbunden mit einem pharmazeutisch verträglichen Verdünnungsmittel oder Trägermittel enthält.

7. Verwendung einer modifizierten Urokinase gemäß jeglichem der Ansprüche 1 bis 3 zur Herstellung einer thrombolytischen Zusammensetzung.

**Revendications**

1. Urokinase modifiée dans laquelle un polymère ou un copolymère hydrosoluble est lié au groupement amino de l'urokinase, caractérisée en ce que l'urokinase modifiée a la formule

R.CO.NH.urokinase

dans laquelle R est le reste d'un monométhoxypolyéthylèneglycol ou d'un monométhoxypolyéthylèneglycol-polypropylèneglycol hydrosoluble ayant un poids de 2000 à 10000 après enlèvement d'un atome d'hydrogène du groupement hydroxy terminal.

2. Urokinase modifiée selon la revendication 1, dans laquelle le reste R a un poids moléculaire moyen de 5000.

3. Urokinase modifiée selon la revendication 1 ou la revendication 2, ayant un poids moléculaire compris entre 100 000, et 150 000 et modifiée jusqu'à une activité résiduelle de 20 à 40%.

4. Urokinase modifiée selon l'une quelconque des revendications 1 à 3, pour son utilisation dans le traitement des thromboses.

5. Procédé de production d'une urokinase modifiée selon l'une quelconque des revendications précédentes, qui consiste à faire réagir un dérivé carbonylimidazole de l'homopolymère ou du copolymère de formule

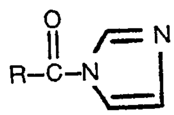 ,

dans laquelle R est tel que défini dans la revendication 1, avec l'urokinase en présence d'une solution tampon à pH 6—11.

6. Composition thrombolytique contenant une urokinase modifiée selon l'une quelconque des revendications 1 à 3 en mélange, ou autrement associée, avec un diluant ou véhicule acceptable sur le plan pharmaceutique.

7. Utilisation d'une urokinase modifiée selon l'une quelconque des revendications 1 à 3, pour la préparation d'une composition thrombolytique.

FIG.1

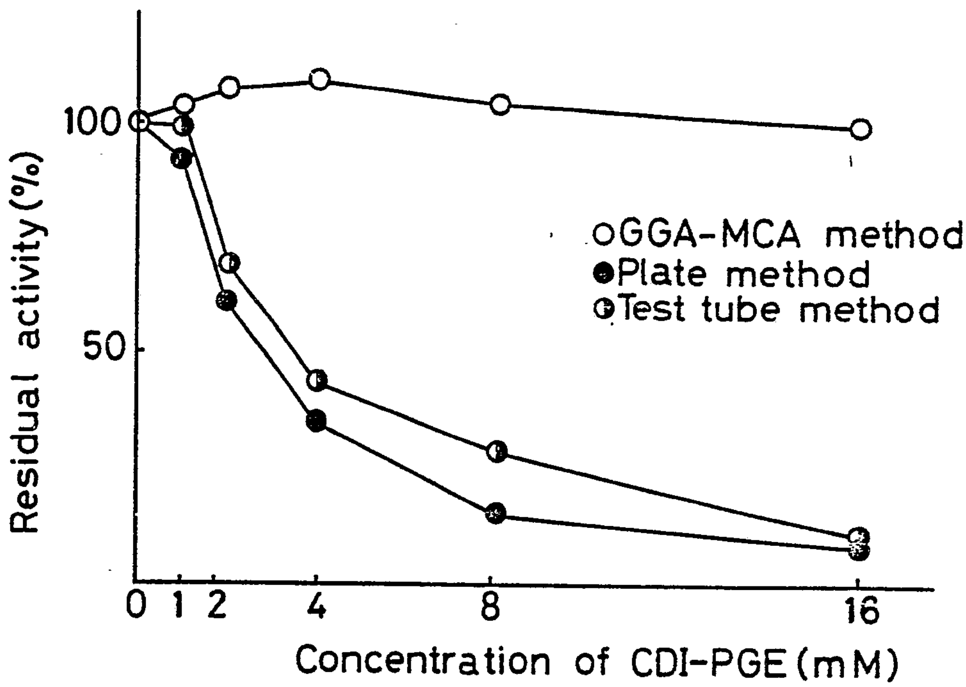
100
50
Residual activity (%)
oGGA-MCA method
●Plate method
◐Test tube method
0 1 2 4 8 16
Concentration of CDI-PGE (mM)

FIG.2

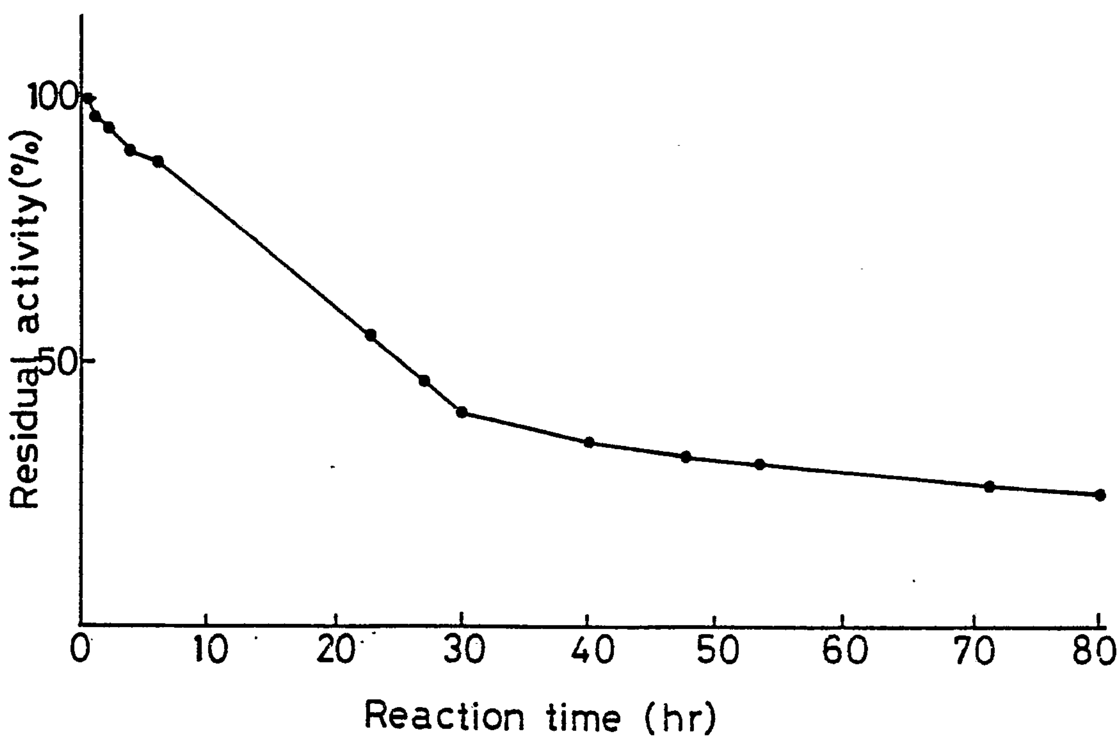
100
50
Residual activity (%)
0 10 20 30 40 50 60 70 80
Reaction time (hr)

FIG. 3

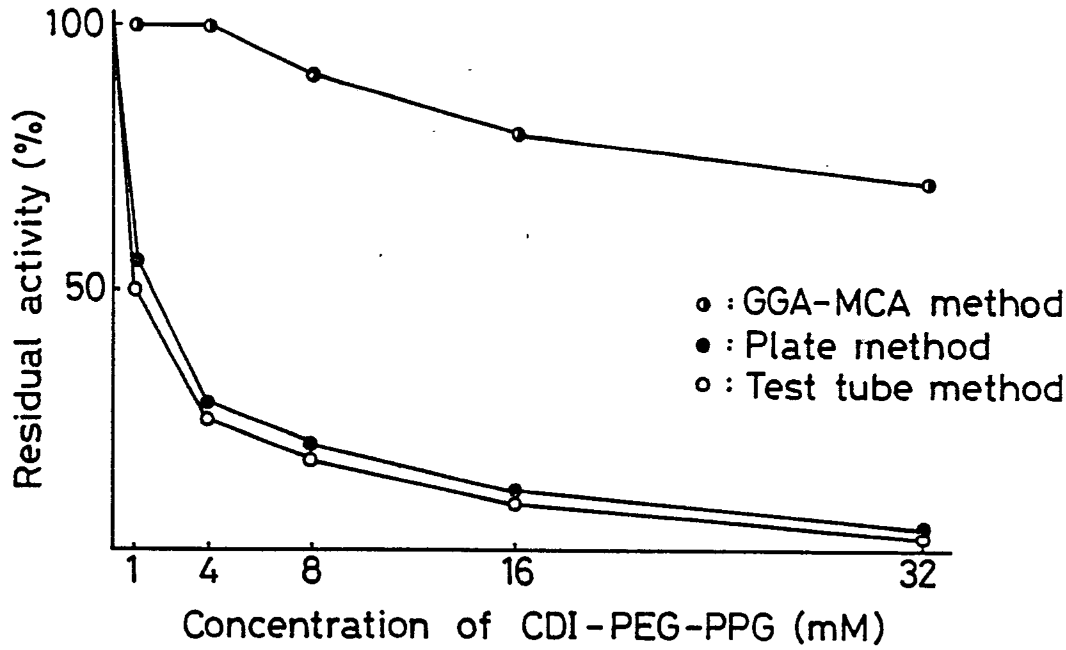
100
50
Residual activity (%)
: GGA-MCA method
: Plate method
: Test tube method
1
4
8
16
32
Concentration of CDI-PEG-PPG (mM)

FIG. 4

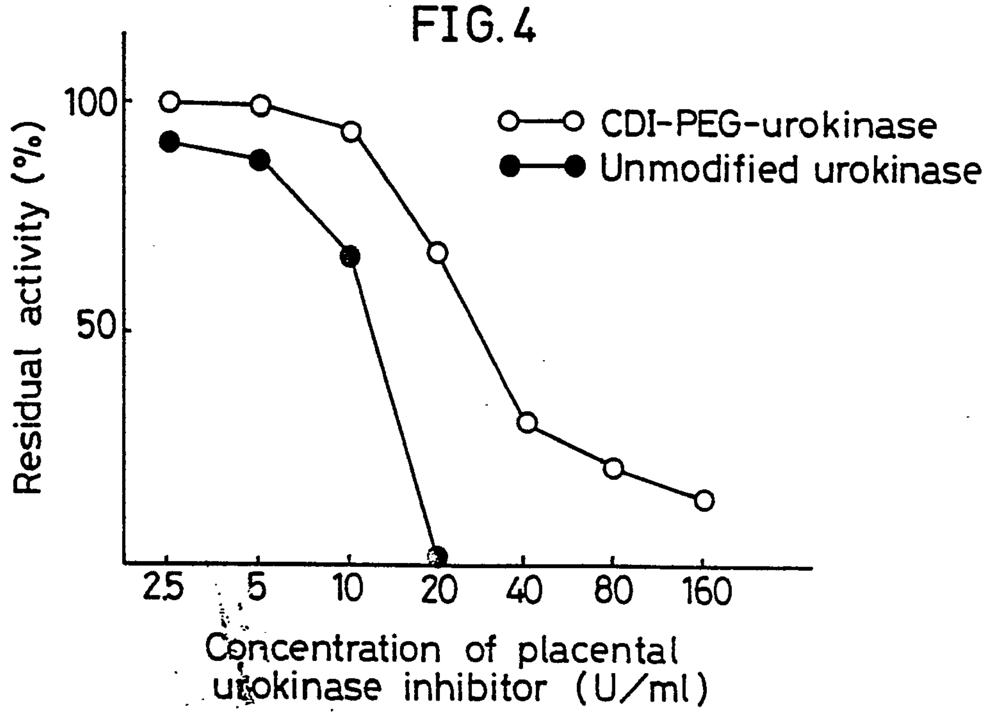
100
50
Residual activity (%)
CDI-PEG-urokinase
Unmodified urokinase
2.5
5
10
20
40
80
160
Concentration of placental
urokinase inhibitor (U/ml)

Fig.5

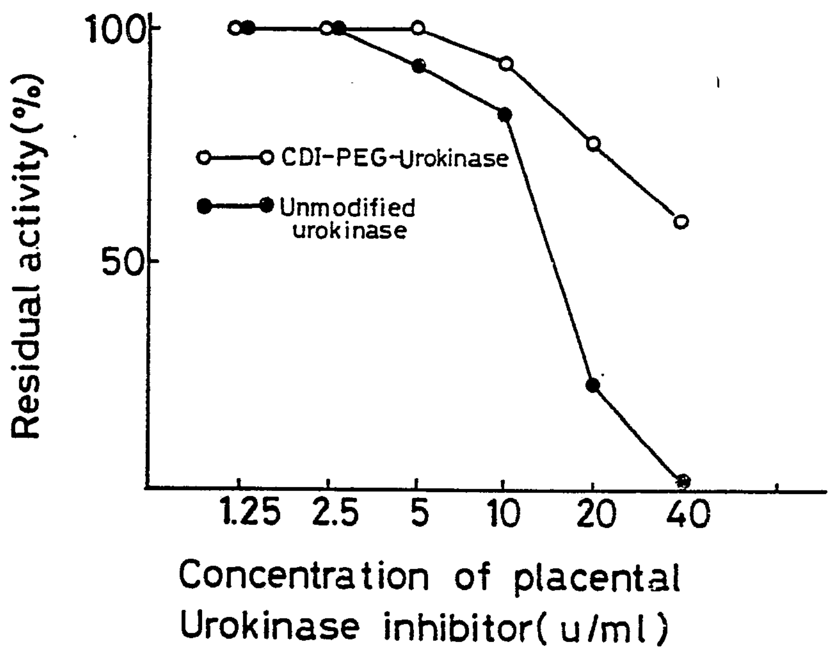
100
50
Residual activity(%)
CDI-PEG-Urokinase
Unmodified urokinase
1.25
2.5
5
10
20
40
Concentration of placental Urokinase inhibitor( u/ml )

FIG.6

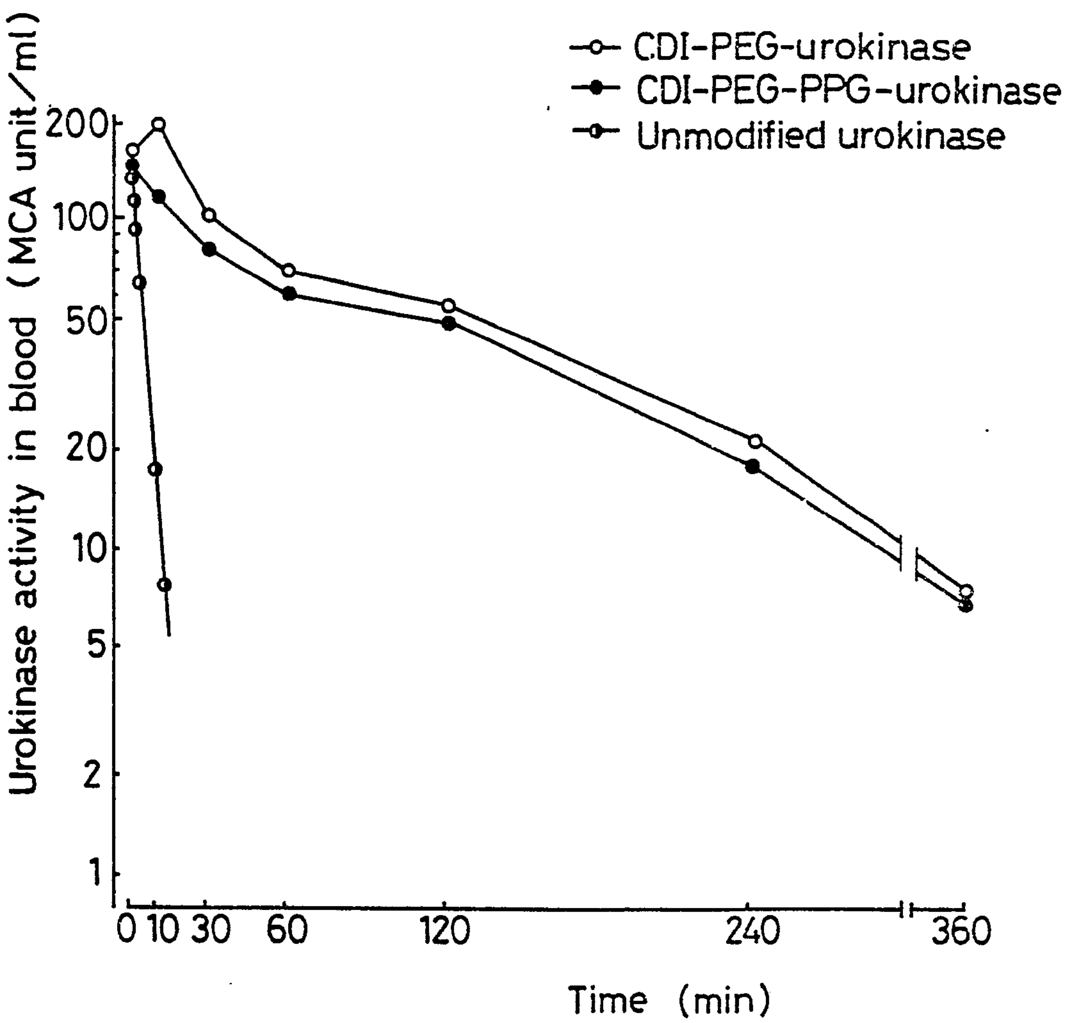
CDI-PEG-urokinase
CDI-PEG-PPG-urokinase
Unmodified urokinase
Urokinase activity in blood (MCA unit/ml)
200
100
50
20
10
5
2
1
0 10 30 60 120 240 360
Time (min)